# EUROPÄISCHE PATENTSCHRIFT

(11) **EP 1 227 808 B1**
(45) Veröffentlichungstag und Bekanntmachung des Hinweises auf die Patenterteilung: **08.10.2003**
(21) Anmeldenummer: 00975995.2
(22) Anmeldetag: 03.11.2000
(51) Int. Cl.: A61K 31/255, A61P 37/06

(54) **VERWENDUNG VON TREOSULFAN ZUR KONDITIONIERUNG VON PATIENTEN VOR KNOCHENMARKTRANSPLANTATION ODER BLUTSTAMMZELLTRANSPLANTATION**
USE OF TREOSULFAN FOR PATIENT CONDITIONING BEFORE BONE MARROW OR BLOOD STEM CELL TRANSPLANTATION
UTILISATION DE TREOSULFANE POUR LE CONDITIONNEMENT DE PATIENTS AVANT UNE GREFFE DE MOELLE OSSEUSE OU UNE GREFFE DE CELLULES SANGUINES SOUCHES

(30) Priorität: 05.11.1999 DE 19953517
(43) Veröffentlichungstag der Anmeldung: 07.08.2002
(73) Patentinhaber: Medac Gesellschaft für klinische Spezialpräparate mbH, D-20354 Hamburg (DE)
(72) Erfinder: BAUMGART, Joachim, 25482 Appen (DE)
(74) Vertreter: UEXKÜLL & STOLBERG
(86) Internationale Anmeldenummer: EP0010871
(87) Internationale Veröffentlichungsnummer: WO01032154

(56) Entgegenhaltungen:
- WO-A-00/45842
- US-A- 5 376 546
- US-A- 5 830 473
- PLOEMACHER R E ET AL: "Treosulfan as an alternative conditioning agent in bone marrow transplantation." BLOOD, Bd. 94, Nr. 10 SUPPL. 1 PART 2, 15. November 1999 (1999-11-15), Seite 324b XP001015768 Forty-first Annual Meeting of the American Society of Hematology;New Orleans, Louisiana, USA; December 3-7, 1999 ISSN: 0006-4971
- VON-PAWEL J ET AL: "Clinical phase II trial of treosulfan in patients with non-resectable non small-cell lung cancer." ONKOLOGIE, Bd. 21, Nr. 4, August 1998 (1998-08), Seiten 316-319, XP001020843 ISSN: 0378-584X
- KALDOR J M ET AL: "LEUKEMIA FOLLOWING CHEMOTHERAPY FOR OVARIAN CANCER" NEW ENGLAND JOURNAL OF MEDICINE, Bd. 322, Nr. 1, 1990, Seiten 1-6, XP001020842 ISSN: 0028-4793
- WESTERHOF G R ET AL: "In vivo and in vitro sensitivity of murine bone marrow hemopoietic progenitors for treosulfan." BLOOD, Bd. 92, Nr. 10 SUPPL. 1 PART 1-2, 15. November 1998 (1998-11-15), Seite 197B XP001023130 40th Annual Meeting of the American Society of Hematology;Miami Beach, Florida, USA; December 4-8, 1998 ISSN: 0006-4971
- BARTH J. ET AL: "[Treo- and busulfan - Similar and yet different]. TREO- UND BUSULFAN: SO AHNLICH UND DOCH VERSCHIEDEN." KRANKENHAUSPHARMAZIE, (2000) 21/11 (581-585). , XP001020800

## Beschreibung

Die vorliegende Erfindung betrifft die Verwendung von Treosulfan zur Herstellung einer pharmazeutischen Zusammensetzung zur Konditionierungstherapie über einen Zeitraum von 2 bis 7 Tagen bei Verabreichung einer gesamtdosis von mindestens 20g/m² Körperoberfläche vor allogener Transplantation von Knochenmark oder hämatopoetischen Stammzellen, wobei Treosulfan entweder mit anderen Chemotherapeutika bzw. immunsupprimierenden Mitteln kombiniert oder als einzig wirksame Substanz angewendet wird.

Patienten werden vor einer allogenen Knochenmark- oder hämatopoetischen Stammzell-Transplantation einer sogenannten Konditionierungstherapie unterzogen, um
a) die eigenen Vorläuferzellen (Stammzellen) der Blutbildung des Empfängers zu eliminieren,
b) eine Immunsuppression des Empfängers zu erreichen, die eine akute Abstoßung des Transpiatates verhindert, und
c) eine zytotoxische Reduktion eventuell noch vorhandener maligne entarteter Zellen im Empfänger zu erreichen.

Als etablierte Mittel der Konditionierung wird z.B. eine Kombination aus Busulfan und Cyclophosphamid verabreicht, bevor die Knochenmark- oder hämatopoetischen Stammzellen des Spenders transplantiert werden.

Ein wesentliches Risiko bei der allogenen Knochenmarktransplantation ist neben der Möglichkeit einer entgleisenden "Graft versus Host-Reaction" (GvHD), d.h. der Aggression der mitübertragenen reaktiven Immunzellen gegen Organe des Empfängers, die vergleichsweise hohe Toxizität der vorangestellten Konditionierungsstherapie. Obwohl die Nebenwirkungen dieser Konditionierung, insbesondere die hohe Busulfan-assoziierte Morbidität und Mortalität ein wesentlicher Nachteil dieser seit langem zum Einsatz kommenden Therapie ist, wurden bislang keine vergleichbar effektiven aber nebenwirkungsärmeren Alternativen entwickelt.

Die Verwendung von Treosulfan zur Konditionierungstherapie wurde von Westerhof et al. beschrieben (Westerhof G.R. et al, Blood 1998, Bd. 92, Nr. 10, S. 197 B). Die einmalige Verabreichung einer Dosis von 9g/m² Körperoberfläche hat Westerhof et al. erlaubt nur eine geringe Anwachsrate von 1,5% der transplantierten Knochenmarkzellen zu erreichen.

Aufgabe der vorliegenden Erfindung ist daher die Bereitstellung einer Konditionierungstherapie bzw. eines Konditionierungsagens, das die im Stand der Technik bekannten Nachteile nicht aufweist.

Die Aufgabe wird erfindungsgemäß durch Verwendung von Treosulfan als Konditionierungsagens gelöst. Insbesondere wird die Aufgabe durch den Gegenstand der beigefügten Patentansprüche gelöst.

Im Rahmen der vorliegenden Erfindung wurde nun überraschenderweise festgestellt, daß die gravierenden Nachteile bezüglich der Toxizität herkömmlicher Konditionierungen vermieden werden können, wenn man Treosulfan zur Herstellung einer pharmazeutischen Zusammensetzung zur Konditionierungstherapie vor allogenen Transplantationen von Knochenmark oder hämatopoetischen Stammzellen (Blutstammzellen, vorzugsweise periphere Blutstammriellen) verwendet.

Bei Treosulfan handelt es sich um L-Treitol-1,4-bis-methansulfonat, ein Dihydroxy-Derivat des Alkylans Busulfan, das erstmals 1961 synthetisiert wurde und bislang nur als konventionell dosiertes Chemotherapeutikum, beispielsweise bei Patientinnen mit Ovarialkarzinom, oral oder intravenös eingesetzt wird.

Erfindungsgemäß wurde festgestellt, daß Treosulfan z.B. anstelle von hochdosiertem Busulfan in vorteilhafter Weise mit Chemotherapeutika und/oder immunsupprimierenden Mitteln, wie Cyclophosphamid, Carboplatin, Thiotepa, Melphalan, Fludarabin, immunsuppresiven Antikörpern oder Ganzkörperbestrahlung, effektiv kombiniert werden kann, wodurch sich die auf Busulfan zurückzuführenden, schwerwiegenden und z.T. mit tödlichem Ausgang verbundenen Nebenwirkungen überwiegend oder vollständig vermeiden lassen. Insbesondere die Busulfan-assoziierten schwerwiegenden Leber,- Lungen-, Nieren- und ZNS-Toxizitäten werden beim Einsatz von Treosulfan selbst in hohen Dosierungen nicht beobachtet.

Ein wesentlicher Vorteil von Treosulfan ist seine im Gegensatz zu Busulfan bestehende hohe Wasserlöslichkeit, die die Zubereitung von Infusionslösungen ermöglicht. Im Gegensatz dazu wird Busulfan aufgrund seiner geringen Löslichkeit üblicherweise in Form von Tabletten verabreicht, die in individuell sehr unterschiedlichem Maße resorbiert werden. Da die orale Verabreichung von Busulfan aufgrund gastro-intestinaler Nebenwirkungen von vielen Patienten ferner nur schlecht vertragen wird, kommt es häufig zum Erbrechen, was eine verläßliche und reproduzierbare Bioverfügbarkeit von z.B. Busulfan im Einzelfall erheblich beeinträchtigt. Intravenös verabreichbare Busulfan-enthaltende Lösungen hingegen sind nur mit Hilfe von Lösungsvermittlern, wie z.B. Dimethylazetamid, erhältlich, die ihrerseits Nebenwirkungen auslösen können.

Im Gegensatz dazu lassen sich mit Treosulfan ohne weiteres Infusionslösungen herstellen. Die intravenöse Applikation ermöglicht eine genaue Wirkstoff-Dosierung und sichere Anwendung. Darüber hinaus hat sich überraschenderweise gezeigt, daß sich mit hochdosiertem Treosulfan allein oder in Kombination mit z.B. Cyclophosphamid oder Fludarabin eine erfolgreiche und sichere Konditionierungstherapie bei gleichzeitig deutlich verminderten Nebenwirkungen durchführen läßt. Die Gesamtdosis des über einen Zeitraum von 2 bis 7 Tagen verabreichten Treosulfans beträgt mindestens 20 g/m² Körperoberfläche und liegt vorzugsweise im Bereich von 20 bis 60 g/m².

Erfindungsgemäß ist es daher möglich, die herkömmliche Konditionierung mit z.B. Busulfan / Cyclophosphamid durch eine Konditionierung unter Verwendung von Treosulfan / Cyclophosphamid oder Treosulfan / Fludarabin zu ersetzen. Dabei werden beispielsweise 3 x 14 bis 16 g Treosulfan/m² Körperoberfläche (entsprechend einer Gesamtdosis von 42 bis 48 g/m²) an drei aufeinanderfolgenden Tagen verabreicht. Anschließend werden 60-100 mg Cyclophosphamid/kg Körpergewicht an den zwei darauffolgenden Tagen infundiert.

Im Rahmen der vorliegenden Erfindung wurde ferner überraschenderweise festgestellt, daß man Treosulfan auch alleine verwenden kann, da Treosulfan in höheren Dosierungen nicht nur primitive Stammzellen, sondern auch die sogenannten "committed stem cells" eliminieren kann. Auf diese Weise können sowohl Busulfan als auch Cyclophosphamid durch Treosulfan allein ersetzt werden, wobei die bei der konventionellen Konditionierungstherapie beobachteten schwerwiegenden und zum Teil tödlichen Nebenwirkungen vob Busulfan und/oder Cyclophosphamid nicht auftreten.

Für die Konditionierungstherapie mit Treosulfan als alleinigem Wirkstoff sind mindestens etwa 20 g Treosulfan/m² Körperoberfläche erforderlich, vorzugsweise werden etwa 20 bis etwa 60 g/m², vorteilhafterweise 30 g/m² und besonders bevorzugt 42 bis 48 g Treosulfan/m² Körperoberfläche als Gesamtdosis für den zu behandelnden Patienten verabreicht. Treosulfan wird über einen Zeitraum von 2 Tagen bis zu 1 Woche (7 Tagen) appliziert. Wird die Konditionierung über einen Zeitraum von 2 bis 7 Tagen (z.B. über 3, 5 oder 7 Tage) durchgeführt, kommen tägliche Infusionen über einen Zeitraum von 0,5 bis 24 Stunden in Betracht. Die Summe der täglichen Einzeldosierungen entspricht der zuvor genannten Treosulfan-Gesamtdosis, wobei die täglich applizierten Wirkstoffmengen untereinander gleich oder verschieden sein können. Denkbar ist auch, die Konditionierung im Rahmen einer über z.B. 3, 5 oder 7 Tage erfolgenden Dauerinfusion durchzuführen.

Die vorliegende Erfindung betrifft somit die Verwendung von Treosulfan zur Herstellung einer pharmazeutischen Zusammensetzung zur Konditionierungstherapie (Konditionierungsagens) vor allogener Transplantation von Knochenmark oder hämatopoetischen Stammzellen, wobei Treosulfan entweder alleine oder zusammen (in Kombination) mit anderen Chemotherapeutika und/oder immunsupprimierenden Mitteln (wie z.B. Cyclophosphamid, Fludarabin, Thiotepa, Melphalan, Carboplatin, immunsuppressiven Antikörpern, Ganzkörperbestrahlung etc.) verabreicht wird. Das heißt, daß man Treosulfan entweder als alleinigen Wirkstoff oder in Kombination mit mindestens einem weiteren chemotherapeutischen Agens und/oder immunsupprimierenden Mitteln zur Herstellung eines Konditionierungsagens bzw. Konditionierungsmedikaments verwendet, wobei die pharmazeutische Zusammensetzung in besonderer Weise zur Konditionierungstherapie bei zusätzlicher Ganzkörperbestrahlung des Patienten geeignet ist.

Bei der Verwendung zur Herstellung der pharmazeutischen Zusammensetzung, die Treosulfan und mindestens ein weiteres chemotherapeutisches Agens und/oder immunsupprimierende Mittel enthält, handelt es sich um die Herstellung eines Kombinationspräparates, das entweder in Form einer alle Wirkstoffe enthaltenden Formulierung oder in einer Verpackungseinheit vorliegt, die die Wirkstoffe in räumlich getrennten Formulierungen (in getrennten Behältern) umfaßt, d.h in Form einer Treosulfan-enthaltenden pharmazeutischen Zusammensetzung und einer (davon räumlich getrennten) mindestens ein chemotherapeutisches Agens und/oder immrinsupprimierende> Mittel enthaltenden pharmazeutische Zusammensetzung. Dementsprechend werden die Wirkstoffe bei den oben genannten Kombinationspräparaten vor der Knochenmartransplantation oder Blutstammzelltransplantation entweder gleichzeitig oder zeitlich versetzt verabreicht.

Die vorliegende Erfindung betrifft daher neben der Verwendung von Treosulfan als alleinigem Wirkstoff zur Herstellung eines Konditionierungsagens auch Kombinationspräparate, die Treosulfan und mindestens ein weiteres chemotherapeutisches Agens und/oder immunsupprimierende Mittel enthalten. Die Einzelheiten zur Verwendung (Verabreichung) von Treosulfan, einschließlich der Kombination mit anderen Wirkstoffen (insbesondere mit Cyclophosphamid), sind bereits oben genannt. Die pharmazeutischen Zusammensetzungen und Kombinationspräparate sind dementsprechend so formuliert und enthalten die jeweils genannten Wirkstoffe in solchen Konzentrationen, daß sie zur Verabreichung der genannten Wirkstoffmengen über die angegebenen Zeiträume geeignet sind.

Verglichen mit Busulfan besitzt Treosulfan trotz identischer DNA-Alkylierungsorte möglicherweise aufgrund seines unterschiedlichen Alylierungsmechanismus eine vergleichbare Wirksamkeit jedoch ein erheblich reduziertes Nebenwirkungspotential. So zeigt Treosulfan eine signifikante Stammzelltoxizität sowohl gegenüber primitiven Stammzellen als auch gegenüber sogenannten "committed stem cells", während es im Gegensatz zu Busulfan ein nur geringes nicht-hämatologisches Toxizitätsprofil und insbesondere keine signifikante Leber-, Nieren-, ZNS- oder Lungentoxizität aufweist.

Aufgrund seiner hohen Wasserlöslichkeit kann Treosulfan leicht intravenös verabreicht werden und zeigt verläßliche und lineare pharmakokinetische Eigenschaften.

Die Erfindung wird nachfolgend anhand von Beispielen beschrieben.

### Beispiele

Es wurde untersucht, ob Treosulfan vergleichbare stammzelltoxische Eigenschaften bezüglich des Knochenmarks wie Busulfan aufweist, wobei beide Wirkstoffe bei einer maximal tolerierten Dosis in einem murinen Knochenmarktransplantationsmodell verabreicht wurden.

Die Empfängermäuse (B6-*Gpi-1*^{*b*}) wurden mit 3 x 2500 mg/kg bzw. 7500 mg/kg (entsprechend 22,5 g/m²) Treosulfan bzw. 50 mg/kg Busulfan behandelt. 24 Stunden nach Verabreichung wurde die Eliminierung der unterschiedlichen hämopoetischen Stammzellen im Wirts-Knochenmark unter Verwendung des *in vitro* Cobble stone area forming cell assays (CAFC; vgl. Ploemacher et al., *Blood* 78 (1991) 2527-2533) ermittelt.

Die tägliche Fraktionierung der verabreichten höheren Treosulfan-Dosen (3 x 2500 mg/kg) führte überraschenderweise zu einer effektiven Eliminierung sowohl der primitiven als auch der sogenannten "committed" Progenitorzellen um 3 bis 5 Zehnerpotenzen.

In fraktionierten Dosen verabreichtes Treosulfan wurde auch in einem H-2-kompatiblen allogenen Knochenmarktransplantationsversuch (C.B10->B6) evaluiert. Dabei muß eine immunologische Abstoßungsreaktion des Transplantates durch die Konditionierung mit z.B. Treosulfan verhindert werden, bevor ein Anwachsen des Transplantates möglich wird. In diesem Fall war Treosulfan allein in der Lage, einen nahezu vollständigen und stabilen Donor-Typ-Chimärismus im Empfänger zu induzieren. Dagegen war Busulfan allein nicht in der Lage, eine effektive Konditionierung der Versuchstiere vor allogener Knochenmarktransplantation zu bewirken. Vielmehr mußten zusätzliche Gaben von z.B. Cyclophosphamid eine ausreiche Immunsuppression bewirken, um eine Transplantatabstoßung zu verhindern und einen kompletten Donor-Typ-Chimärismus zu induzieren.

## Patentansprüche

1. Verwendung von Treosulfan zur Herstellung einer pharmazeutischen Zusammensetzung zur Konditionierungstherapie wobei die Zusammensetzung zur Verabreichung über einen Zeitraum von 2 bis 7 Tagen bei einer Gesamtdosis von mindestens 20 g/m² Körperoberfläche vor allogener Transplantation von Knochenmark oder hämatopoetischen Stammzellen bestimmt ist.

2. Verwendung nach Anspruch 1, **dadurch gekennzeichnet, daß** die Gesamtdosis 20 bis 60 g/m² Körperoberfläche beträgt.

3. Verwendung nach Anspruch 2, **dadurch gekennzeichnet, daß** die Gesamtdosis 30 g/m² Körperoberfläche beträgt.

4. Verwendung nach Anspruch 2, **dadurch gekennzeichnet, daß** die *Gesamtdosis* 42 bis 48 g/m² Körperoberfläche beträgt.

5. Verwendung nach den Ansprüchen 1 bis 4, **dadurch gekennzeichnet, daß** die Konditionierungstherapie über einen Zeitraum von 3, 5 oder 7 Tagen erfolgt.

6. Verwendung nach den Ansprüchen 1 bis 4, **dadurch gekennzeichnet, daß** die Konditionierungstherapie in Form einer Dauerinfusion erfolgt.

7. Verwendung nach den Ansprüchen 1 bis 4, **dadurch gekennzeichnet, daß** die Konditionierungstherapie in Form täglicher Einzeldosen erfolgt.

8. Verwendung nach Anspruch 4, **dadurch gekennzeichnet, daß** man die pharmazeutische Zusammensetzung so formuliert, daß sie zur Infusion von Treosulfan über einen Zeitraum von 2 bis 7 Tagen jeweils täglich über einen Zeitraum von 0,5 bis 24 Stunden geeignet ist.

9. Verwendung nach den Ansprüchen 1 bis 8, **dadurch gekennzeichnet, daß** man Treosulfan zusammen mit mindestens einem weiteren chemotherapeutischen Agens und/oder immunsupprimierenden Mitteln verwendet.

10. Verwendung nach Anspruch 9, **dadurch gekennzeichnet, daß** die pharmazeutische Zusammensetzung ein Kombinationspräparat ist.

11. Verwendung nach Anspruch 10, **dadurch gekennzeichnet, daß** das chemotherapeutische Agens ein Cytostatikum ist.

12. Verwendung nach Anspruch 11, **dadurch gekennzeichnet, daß** das Cytostatikum aus der Gruppe bestehend aus Cyclophosphamid, Thiotepa, Melphalan, Carboplatin oder Fludarabin ausgewählt ist.

13. Verwendung nach den Ansprüchen 9 bis 12, **dadurch gekennzeichnet, daß** die immunsupprimierenden Mittel immunsuppressive Antikörper sind.

14. Verwendung nach den Ansprüchen 9 bis 12 zur Konditionierungstherapie bei zusätzlicher Ganzkörperbestrahlung des Patienten.

15. Verwendung nach Anspruch 12, **dadurch gekennzeichnet, daß** das Cytostatikum Cyclophosphamid ist und man die pharmazeutische Zusammensetzung so formuliert, daß sie zur Verabreichung von je 14 bis 16 g Treosulfan/m² Körperoberfläche an 3 aufeinanderfolgenden Tagen und von je 60 bis 100 mg Cyclophosphamid/kg Körpergewicht an den 2 darauffolgenden Tagen geeignet ist.

## Claims

1. Use of treosulfan for manufacturing a pharmaceutical preparation for conditioning therapy whereby the preparation is intended for administration over a period of between 2 and 7 days with a total dose of at least 20 g/m² body surface area before allogenic transplantation of bone marrow or haematopoietic stem cells.

2. Use according to Claim 1, **characterised in that** the total dose is between 20 and 60 g/m² body surface area.

3. Use according to Claim 2, **characterised in that** the total dose is 30 g/m² body surface area.

4. Use according to Claim 2, **characterised in that** the total dose is between 42 and 48 g/m² body surface area.

5. Use according to Claims 1 to 4, **characterised in that** the conditioning therapy takes place over a period of 3, 5 or 7 days.

6. Use according to Claims 1 to 4, **characterised in that** the conditioning therapy takes place in the form of a continuous infusion.

7. Use according to Claims 1 to 4, **characterised in that** the conditioning therapy takes place in the form of daily single doses.

8. Use according to Claim 4, **characterised in that** the pharmaceutical preparation is so formulated that it is suitable for infusion of treosulfan over a period of between 2 and 7 days, in each case daily over a period of between 0.5 and 24 hours.

9. Use according to Claims 1 to 8, **characterised in that** treosulfan is used together with at least one further chemotherapeutic agent and/or immunosuppressant means.

10. Use according to Claim 9, **characterised in that** the pharmaceutical preparation is a compound preparation.

11. Use according to Claim 10, **characterised in that** the chemotherapeutic agent is a cytostatic agent.

12. Use according to Claim 11, **characterised in that** the cytostatic agent is selected from the group comprising cyclophosphamide, thiotepa, melphalan, carboplatin or fludarabine.

13. Use according to Claims 9 to 12, **characterised in that** the immunosuppressant agents are immunosuppressive antibodies.

14. Use according to Claims 9 to 12 for conditioning therapy with additional whole body irradiation of the patient.

15. Use according to Claim 12, **characterised in that** the cytostatic agent is cyclophosphamide and the pharmaceutical preparation is so formulated that it is suitable for administration of between 14 and 16 g treosulfan/m² body surface area on each of three successive days and of between 60 and 100 mg cyclophosphamide/kg body weight on each of the two following days.

## Revendications

1. Utilisation de tréosulfane pour la préparation d'une composition pharmaceutique pour une thérapie de conditionnement, où la composition est identifiée pour une administration sur une période de 2 à 7 jours à une dose totale d'au moins 20 g/m² de surface corporelle avant une transplantation allogène de moelle osseuse ou de cellules souches hématopoïétiques.

2. Utilisation selon la revendication 1, **caractérisée en ce que** la dose totale se situe dans l'intervalle allant de 20 à 60 g/m² de surface corporelle.

3. Utilisation selon la revendication 2, **caractérisée en ce que** la dose totale s'élève à 30 g/m² de surface corporelle.

4. Utilisation selon la revendication 2, **caractérisée en ce que** la dose totale se situe dans l'intervalle allant de 42 à 48 g/m² de surface corporelle.

5. Utilisation selon les revendications 1 à 4, **caractérisée en ce que** la thérapie de conditionnement est réalisée sur une période de 3, 5 ou 7 jours.

6. Utilisation selon les revendications 1 à 4, **caractérisée en ce que** la thérapie de conditionnement est réalisée sous forme d'une perfusion.

7. Utilisation selon les revendications 1 à 4 , **caractérisée en ce que** la thérapie de conditionnement est réalisée sous la forme d'une dose unique quotidienne.

8. Utilisation selon la revendication 4, **caractérisée en ce que** l'on formule la composition pharmaceutique de sorte qu'elle soit appropriée pour l'injection de tréosulfane sur une période de 2 à 7 jours, chaque fois quotidiennement, sur une période de 0,5 à 24 heures.

9. Utilisation selon les revendications 1 à 8 , **caractérisée en ce que** l'on utilise le tréosulfane en combinaison avec au moins un autre agent chimiothérapeutique et/ou agent immunosuppresseur.

10. Utilisation selon la revendication 9, **caractérisée en ce que** la composition pharmaceutique est une préparation combinée.

11. Utilisation selon la revendication 10, **caractérisée en ce que** l'agent chimiothérapeutique est un cytostatique.

12. Utilisation selon la revendication 11, **caractérisée en ce que** le cytostatique est choisi parmi le groupe consistant en le cyclophosphamide, le thiotépa, le melphalan, le carboplatine ou la fludarabine.

13. Utilisation selon les revendications 9 à 12, **caractérisée en ce** l'agent inununosuppresseur est un anticorps immunosuppresseur.

14. Utilisation selon les revendications 9 à 12, pour la thérapie de conditionnement lors d'une irradiation de tout le corps du patient.

15. Utilisation selon la revendication 12, **caractérisée en ce que** le cytostatique est le cyclophosphamide et **en ce que** l'on formule la composition pharmaceutique de sorte qu'elle soit appropriée pour l'administration de chaque fois, de 14 à 16g de tréosulfane/m² de surface corporelle sur 3 jours successifs et chaque fois, de 60 à 100 mg de cyclophosphamide/kg de poids de corps sur les 2 jours suivants.
